# EUROPEAN PATENT SPECIFICATION

(11) **EP 4 147 736 B1**
(45) Date of publication and mention of the grant of the patent: **28.10.2026**
(21) Application number: 22195611.3
(22) Date of filing: 14.09.2022
(51) Int. Cl.: A61M 5/142, A61M 5/168

(54) **SYSTEM AND METHOD FOR DETECTING A LEAKING OCCLUDER VALVE**
SYSTEM UND VERFAHREN ZUR ERKENNUNG EINES LECKENDEN OKKLUSIONSVENTILS
SYSTÈME ET PROCÉDÉ DE DÉTECTION D'UNE SOUPAPE D'OCCLUSION DE FUITE

(30) Priority: 14.09.2021 US 202163244206 P
(43) Date of publication of application: 15.03.2023
(73) Proprietor: CareFusion 303, Inc., San Diego, CA 92130 (US)
(72) Inventor: ABAL, Daniel M., San Diego, CA 92130 (US); SUBRAMANIAN, Ramkumar, San Diego, CA 92130 (US)
(74) Representative: Epping - Hermann - Fischer

(56) References cited:
- US-A1- 2019 151 571
- US-A1- 2020 282 138
- US-B2- 7 311 693

## Description

### BACKGROUND

Large volume pumps (LVPs), e.g., pumps delivering 100 mL or more of fluid from a single container, may be subject to maintenance for various failures over time. One potential mode of failure includes incomplete occlusion of the intravenous (IV) tubing by the pump mechanism. For example, the fingers that pinch the tubing to drive fluid within the tubing may not fully compress the tubing or, in some instances, may over-compress the tubing. In the over-compression case, the pump may cause damage to the tubing by placing it under unexpected stress.

Document US 2019/151571 A1 discloses a medical fluid infusion system that includes a pump system configured to deliver a fluid drug to a patient. The system includes a pump, such as a piston pump, for driving fluid to a patient. One or more pressure sensors are configured to measure and detect changes in fluid pressure in a fluid flow line of the infusion system. The documents US 7 311 693 B2 and US 2020/282138 A1 disclose further infusion pump or drug delivery devices.

To resolve incomplete occlusion of an IV line, current methods include running a flow rate accuracy test. However, these tests typically measure the response of the entire system. As such, any deviations in the flow rate can be the result of one or many factors such as inaccurate motor speed, restrictive fitments, and testing setup issues and the like. Consequently, these tests can lead to extensive troubleshooting to replace parts until the root cause of the problem is identified. This added workload represents time and cost wasted to determine a cause. Currently there are no adequate test systems, devices, or methods to identify leaking valves or fingers quickly and efficiently.

### SUMMARY

There is a need to verify the proper operation of LVP infusion pumps with regards to leakage of the occluder valves in pumps with multiple occlusion valves and fingers, as well as leaking in a pump mechanism. Accordingly, the subject technology provides an apparatus and method for efficiently identifying occluder valves that are leaking as set out in the appended set of claims.

According to various implementations, a method includes activating at least one occluder element of an infusion pump to compress a fluid tubing filled with a fluid to isolate the fluid in an upstream portion of the fluid tubing from a downstream portion of the tubing; and while the fluid tubing is compressed by the occluder element: causing a pumping element of the infusion pump to compress an intermediate portion of the tubing, between the downstream portion and the upstream portion, to cause a pressure increase within the fluid tubing; and determining, using at least one pressure sensor, whether the pressure increase is present in a portion of the fluid tubing on a side, of the activated at least one occluder element, opposite the intermediate portion of the tubing.

According to various implementations, an infusion pump comprises a pumping mechanism comprising at least one occluder element configured to compress a fluid tubing filled with a fluid to isolate the fluid in an upstream portion of the fluid tubing from a downstream portion of the fluid tubing when the fluid tubing is received by the infusion pump; a pumping element configured to compress an intermediate portion of the tubing, between the downstream portion and the upstream portion; at least one pressure sensor; and a processor configured to: activate the at least one occluder element of an infusion pump to compress a fluid tubing filled to isolate the fluid in an upstream portion of the tubing from a downstream portion of the tubing; and while the fluid tubing is compressed by the occluder element: cause the pumping element to compress an intermediate portion of the tubing, between the downstream portion and the upstream portion, to cause a pressure increase within the fluid tubing; and determine, using the at least one pressure sensor, whether the pressure increase is present in a portion of the fluid tubing on a side, of the activated at least one occluder element, opposite the intermediate portion of the tubing.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Description of Implementations below, in conjunction with the following drawings. Like reference numerals refer to corresponding parts throughout the figures and description.
FIG. 1 depicts a perspective view of an example infusion device showing an infusion set in place within the infusion device, according to various aspects of the subject technology.
FIG. 2A depicts an example pumping mechanism of an infusion pump including two occluder valves, according to various aspects of the subject technology. FIG. 2B depicts an example delivery pattern for the example pumping mechanism of FIG. 2A.
FIG. 3 depicts an example occluder valve, including an occluder element, according to various aspects of the subject technology.
FIGS. 4A and 4B depict an example cam with a one-way bearing clutch for testing a pumping element, according to various aspects of the subject technology.
FIGS. 5A and 5B depict examples of rotation and engagement of the test cam 142, including the one way bearing clutch, for testing a pumping element, according to various aspects of the subject technology.
FIG. 6A depicts an example cam phase diagram for testing a pumping element, according to various aspects of the subject technology.
FIG. 6B depicts an example sensor positioning for testing a pumping element, according to various aspects of the subject technology.
FIG. 7 depicts an example process for testing a pumping element, according to aspects of the subject technology.
FIG. 8 is a conceptual diagram illustrating an example electronic system for testing a pumping element, according to aspects of the subject technology.

### DESCRIPTION

Reference will now be made to implementations, examples of which are illustrated in the accompanying drawings. In the following description, numerous specific details are set forth, in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

A broken platen or broken boss (e.g., door releasing boss or pushing boss) may lead to an unregulated flow scenario where the occluder mechanism does not completely pinch the pumping segment and creating a deviation from the set flow rate. The subject technology provides a system and method that can be utilized during pump maintenance. According to some implementations, the subject technology uses an electric current within the IV set to determine whether one or more occluder valves of the pump are faulty. According to various implementations, the subject technology pressurizes the pumping chamber when both upper and lower occluders are closed, and determines if one or both of the occluders are leaking by detecting the pressure increase in the upper and lower pressure sensors. According to various implementations, the pressure is produced by a special cam that engages the main pumping finger to displace it a certain amount which would cause the pressure increase.

According to various implementations of the subject technology, a fluid tubing is loaded in an infusion pump and primed with a fluid. Upper and lower occluder elements of an infusion pump are activated to compress the fluid tubing to isolate the fluid in an upstream portion of the tubing from a downstream portion of the tubing. While the fluid tubing is compressed by the occluder elements, a pumping element of the infusion pump is activated to compress an intermediate portion of the tubing, between the downstream portion and the upstream portion, to cause a pressure increase within the fluid tubing. Using at least one pressure sensor, a determination is made as to whether the pressure increase is present in a portion of the fluid tubing on a side, of an activated occluder element, opposite the intermediate portion of the tubing.

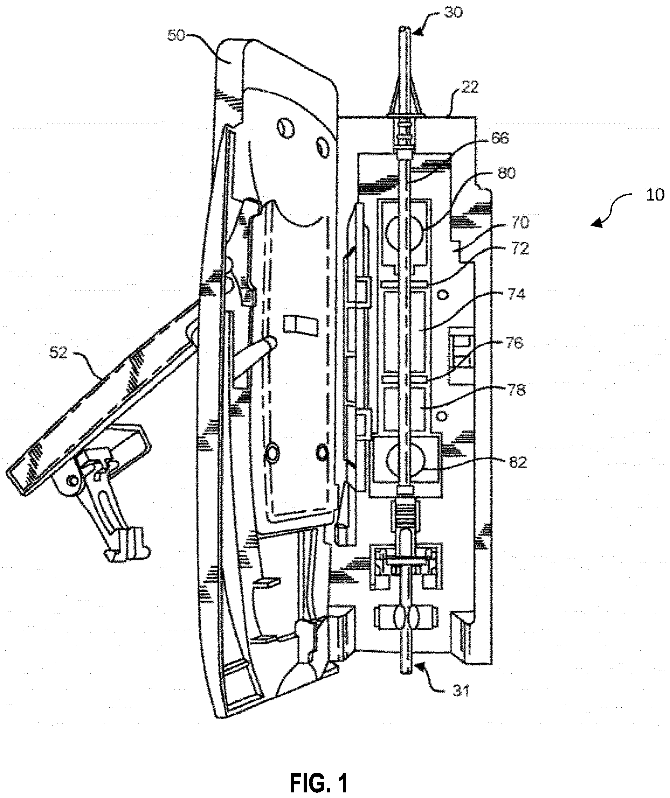

FIG. 1 depicts a perspective view of an example infusion device showing an infusion set in place within the infusion device, according to various aspects of the subject technology. An infusion system for parenteral infusion of a medical fluid to a patient comprises a pump unit, a major part of which comprises a housing which accommodates, in manner known per se, a cam system (not shown) controlling a plurality of fingers of a pumping mechanism, an electric motor and associated gearing, driving said cam mechanism, and further accommodates electronic control and processing circuitry for controlling such motor and processing signals from pressure sensors etc. provided on the unit. The pump unit, as shown, may also comprise an electronically operated display, an alarm light, an input keyboard or other manually operated controls, all in manner known per se.

As shown in FIG. 1, an infusion pump 10 is shown in perspective view with the front door 50 open, showing the upstream fluid line 30 and downstream fluid line 31 in operative engagement with the pump 10. The infusion pump 10 directly acts on a tube 66 that connects the upstream fluid line 30 to the downstream fluid line 31 to form a continuous fluid conduit, extending from a respective fluid supply to a patient, through which fluid is acted upon by the pump to move fluid downstream to the patient. Specifically, a pumping mechanism 70 acts as the flow control device of the pump to move fluid though the conduit. The depicted references 30, 31, 66 may be used to describe herein portions of one continuous fluid line or, in some implementations, may individually describe portions that are fluidly connected together to form a continuous fluid line. The upstream and downstream fluid lines and/or tube 30, 31, 66 may also be coupled to a pump cassette or cartridge that is configured to be coupled to the pump 10, such as the type described in co-pending U.S. patent application Ser. No. 13/827,775.

The type of pumping mechanism may vary and may be for example, a multiple finger pumping mechanism. For example, the pumping mechanism may be of the "four finger" type and includes an upstream occluding element or finger 72, a primary pumping element or finger 74, a downstream occluding element or finger 76, and a secondary pumping element or finger 78. The "four finger" pumping mechanism and mechanisms used in other linear peristaltic pumps operate by sequentially pressing on a segment of the fluid conduit by means of the cam-following pumping elements (e.g., pumping fingers and valve fingers) 72, 74, 76, and 78, which make four finger pump assembly. The pressure is applied in sequential locations of the conduit, beginning at the upstream end of the pumping mechanism, and working toward the downstream end. At least one finger is always pressing hard enough to occlude the conduit. As a practical matter, one finger does not retract from occluding the tubing until the next one in sequence has already occluded the tubing; thus, at no time is there a direct fluid path from the fluid supply to the patient. The operation of peristaltic pumps including four finger pumps is well known to those skilled in the art and no further operational details are provided here.

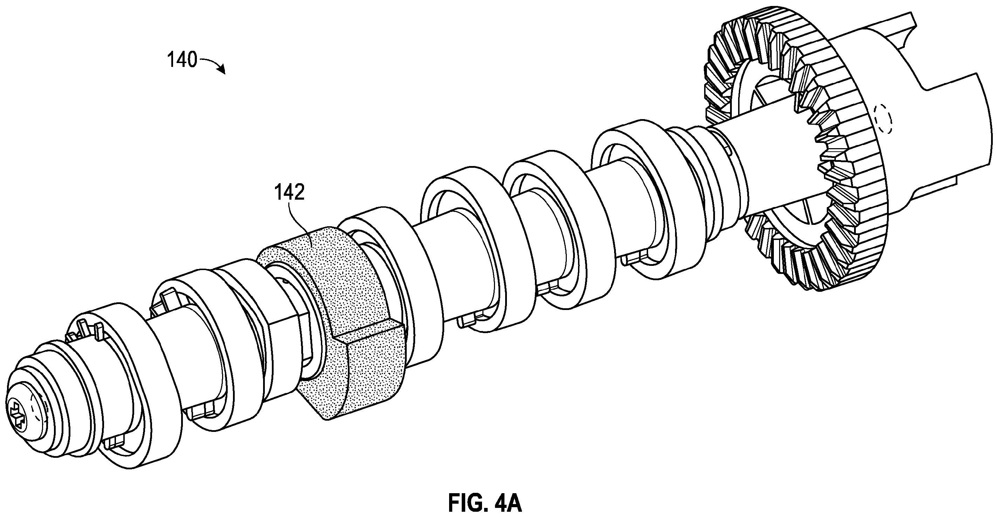

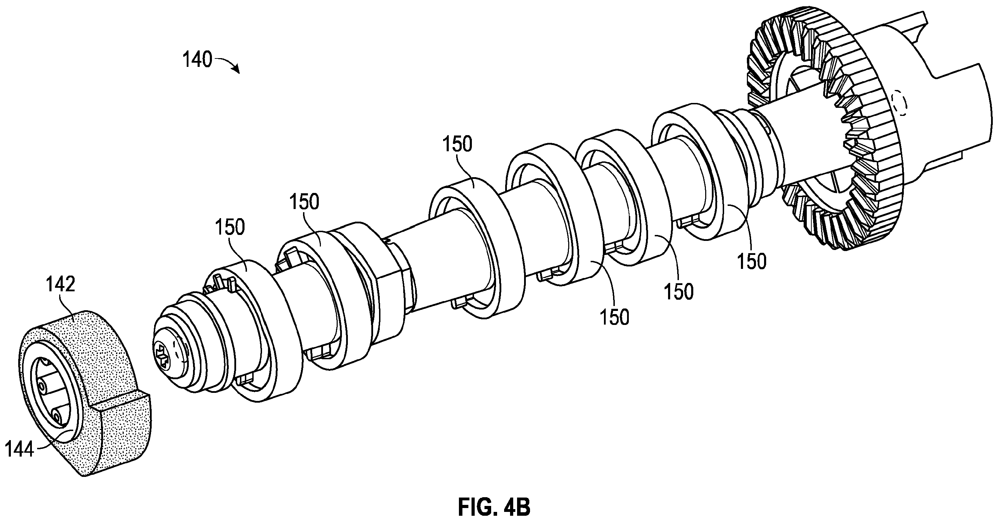

In some implementations, intermediate pumping mechanism 74 may include multiple intermediate elements or fingers that sequentially activate according to a positioning of cam lobes on a camshaft to apply a downward compression against tubing 66, to move the fluid in the tubing 66 downstream. As will be described further, with regard to FIGS. 4A and 4B, one or more of these intermediate fingers may be associated with a test cam that may be selectively activated to test performance of the four finger pump assembly.

FIG. 1 further shows a downstream pressure sensor 82 included in the pump 10 embodiment at a downstream location with respect to the pumping mechanism. The downstream pressure sensor 82 is mounted to the flow control device 70 and is located adjacent and downstream in relation to the flow control device. The downstream pressure sensor is located downstream from the flow control device, that is, at a location between the patient and the flow control device, so that the connection of the correct fluid supply with the correct pump may be verified before any fluid is pumped to the patient.

An upstream pressure sensor 80 may also be included in the pump 10. The upstream pressure sensor is assigned to the flow control device or pumping mechanism 70 and, in this example, is further provided as an integral part of the pump 10. It is mounted to the flow control device 70 and is located adjacent and upstream in relation to the flow control device. The upstream pressure sensor is located upstream from the flow control device, that is, at a location between a fluid supply and the flow control device, so that the connection of the correct fluid supply with the correct pump may be verified before any fluid is pumped to the patient.

As shown in FIG. 1, the face plate 50 which may be opened to reveal the internal loading mechanism for an infusion set. Within the housing of the infusion device (e.g., behind the door or face place), the infusion device includes a pumping segment including a group of serially-aligned pumping elements configured to compress an elongated compressible channel of an infusion set 66, when loaded within the pumping segment. The pumping segment includes a group of serially-aligned pumping elements (e.g., occluders and/or pumping finger(s)) configured to compress the elongated compressible channel (e.g., an IV tubing segment) loaded within the pumping segment.

The infusion set includes an intermediate section of the resiliently compressible tubing 66, for example of silicone rubber and, in some implementations, upper and/or lower fittings which each tubing section may be connected respectively with a respective upper line 30 and with the lower line 31. In use, each upper line 30 extends upwardly to a source of the medical fluid to be administered whilst the lower line 31 extends from the infusion pump to an infusion needle or the like inserted into the patient. In use, the infusion set 66 is extended across the face or deck of the pump unit so that its fittings (not labeled) are received in respective brackets respectively and so that the tubing segment extends over the four finger pump assembly 72, 74, 76, 78. In the depicted example, the infusion set is fitted in place in this fashion whilst the door 50 is in the open position. After the infusion line has been so fitted, the door 50 may be moved to the closed position and is secured by a catch 52 which may include a lever mounted on the outer edge of the door.

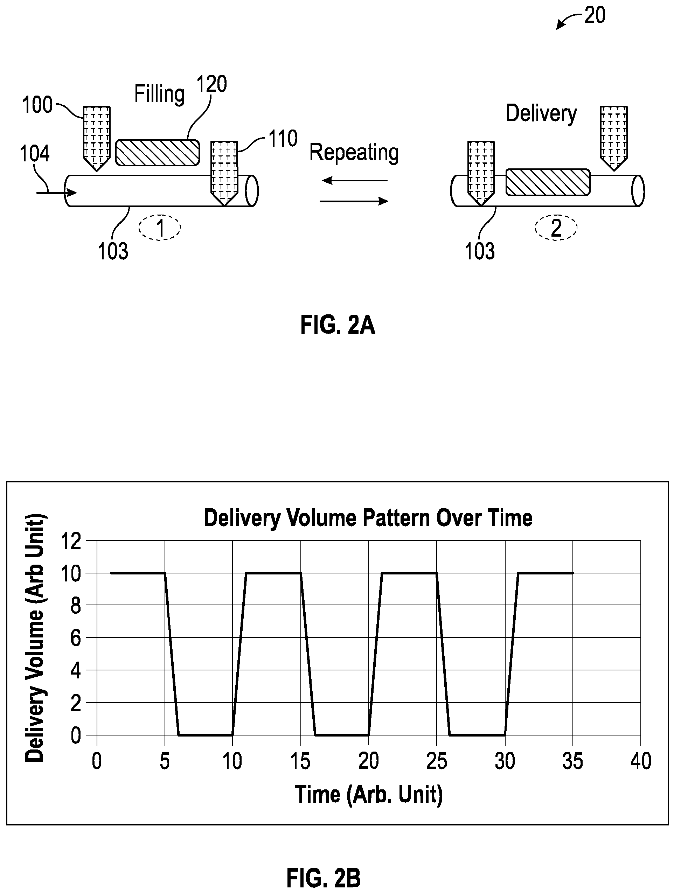

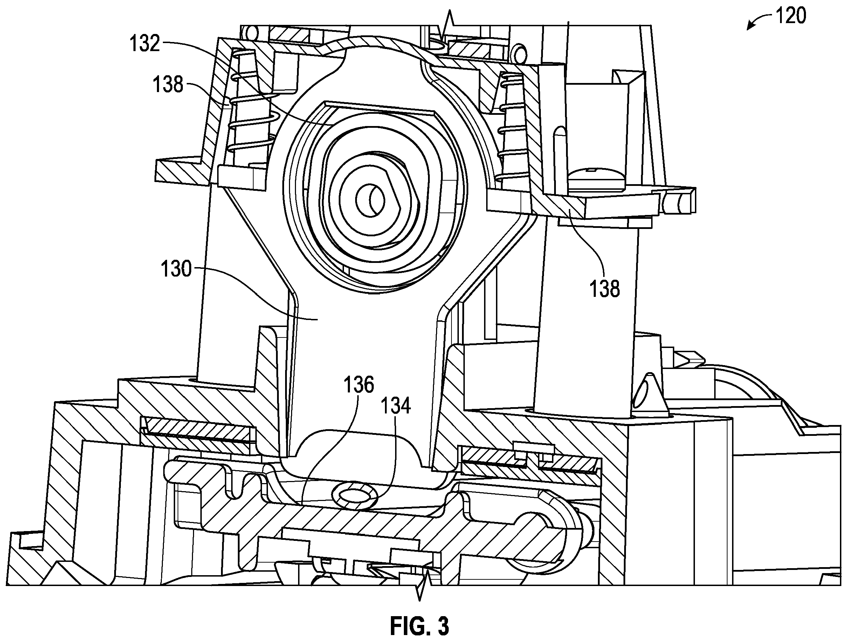

The four finger pump assembly 72, 74, 76, 78 includes respective fingers that are moveable by a cam system (See FIGS. 3-5) inwards and outwards from the face or deck of the pump to compress a respective tubing segment against a counter surface or anvil to propel fluid within the infusion line. In order to make it easier to maintain sterile conditions, these fingers may be covered by a thin flexible membrane, (not shown), sealed at its edges with respect to the deck. The fingers of the pump assembly periodically press the flexible resilient tubing against the counter surface which may be configured on an opposite side, for example, on an inner portion of the door 50. In operation, assuming the fluid is to be propelled downwards, as viewed in FIGS. 2 and, along the infusion line.

FIG. 2A depicts an example pumping mechanism 20 of an infusion device 10 including two occluder valves 100, 110 (e.g., pumping elements 72, 76), according to various aspects of the subject technology. A typical medical pump for IV infusion delivery has two occluders, a first occluder 100 located upstream and a second occluder 110 located downstream, with a plunger 120 (e.g., pumping element 74) in between. The occluders and plunger coordinate with each other in programmable, sequential steps, controlled by a cam shaft to have two phases: 1) a filling phase, and 2) a delivery phase. The occluders move fluid in a tubing 103 by sequentially compressing the tubing, thereby causing a flow in a direction 104 according to the particular compression sequence of the occluders.

During the medication infusion process, in the filling phase, the upstream occluder 100 lifts to suck the medication into the tubing segment, which creates a pause, followed by the delivery phase to push the fluid out. These sequences can repeat through multiple cycles. To specify, when the plunger of a single plunger/tubing design is lifted from the tubing segment during the filling phase, there will be a disruption in the continuous infusion process. As a result of using this design, the medication delivery will behave with a pulse patten as shown in FIG. 2B.

FIG. 3 depicts an example occluder valve 120, including an occluder element 130, according to various aspects of the subject technology. According to various implementations, there are two occluder elements 130; one for the upper occluder 72, and one for the lower occluder 76 of FIG. 1. Occluder element 130 is configured to move according to a cam motion of a cam 132 to apply a periodic compression to a flexible infusion line 134 when the flexible infusion line is placed between the occluder element 130 and a plate assembly 136, also termed "platen". In this regard, the cam motion oscillates the occluder element 130 to occlude a fluid within the flexible infusion line by periodically compressing the flexible infusion line. Compression springs 139 apply a constant force to the occluder element 130, forcing it against the plate assembly 136, while the cam 132 applies a force at predetermined intervals in an opposite direction, moving the lower portion of the occluder element responsible for compressing the infusion line 134 away from the plate assembly. Each cam 132 may be elliptical in shape and may rotate on an axis off center of the ellipse.

In the example of FIG. 3, the occluder element 130 is shown in the top dead center position (top position) with the stroke of the cam is fully upward and with the occluder element furthest apart from the plate assembly 136. A normal occluder element 130 compresses a tubing with a predetermined tolerance. That is, the occluder valve has a certain gap tolerance, or threshold distance between the platen 136 and the occluder element 130 when it is fully extended whereby the occluder element 130 will still fully compress the tubing 134. The platen 136 may be capable of moving towards and away with respect to the valve structure, and may be mechanically connected to the door mechanism of the door 50 shown in FIG. 1. In this regard, the platen 136 may move away from the occluder element and an upper frame 139 of the valve 120 when the door 30 is opened, and move to lock in place as depicted in FIG. 3 when the door 50 is closed.

FIGS. 4A and 4B depict an example camshaft with a one-way bearing clutch for testing a pumping element, according to various aspects of the subject technology. As described previously, upper and lower occluders 72, 76, and intermediate finger(s) 74 may each be movable by a cam. Integrally connected onto camshaft 140 at predetermined locations along the axis of the camshaft 140 may be a series of cam lobes 150. As will be appreciated by those skilled in the pertinent art, cam lobes 150 are eccentrically mounted on camshaft 140 in a pattern along the axis of camshaft 140 and are engaged with a respective pump element in a manner which, in some implementations, may create a wave-like movement of pumping elements when the camshaft 140 is rotated.

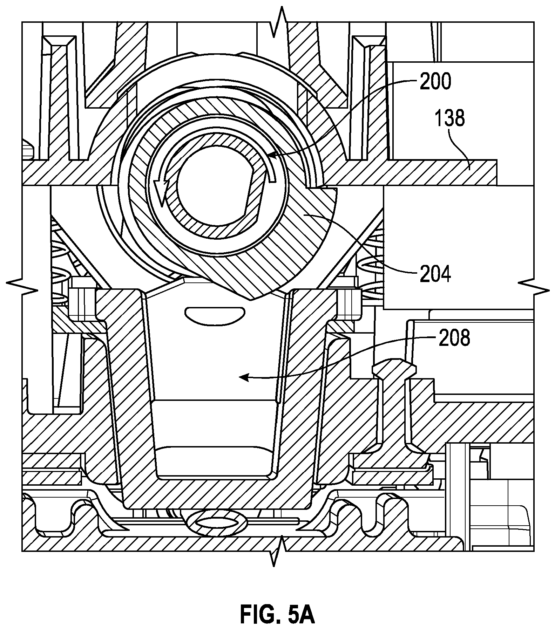

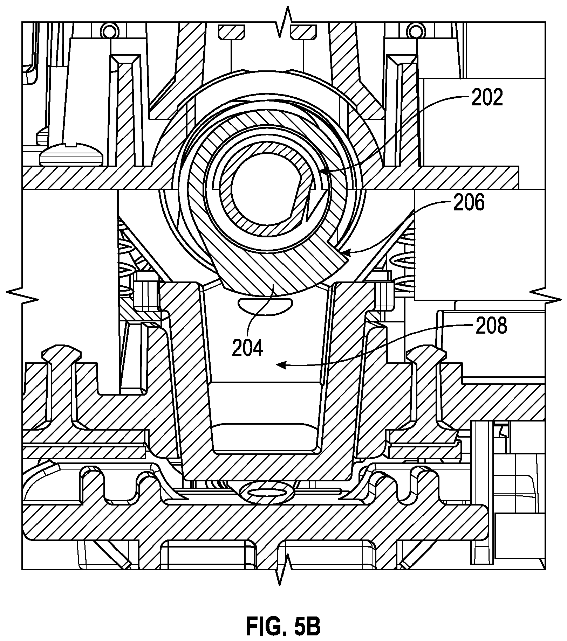

Once the fluid tubing of an infusion set 66 is loaded in infusion pump 10 and engaged with the four finger pump assembly, the door 50 is closed. The closure of door 50 causes the platen to contact a section of the tubing and enclose the section between the platen and a tube support (not shown) for supporting the tube in substantially a flat linear orientation. Activation of a stepper motor rotates a drive shaft causing a drive pulley to rotate the camshaft. The actual positioning of camshaft 140 may be represented by respective camshaft rotation angles θ, as depicted in FIG. 5. Revolution of camshaft 140 will cause the series of cam lobes 150 to reciprocate the pumping elements in a direction substantially perpendicular to the axis of, for example, the intermediate section of resiliently compressible tubing. Due to the configuration of cam lobes 150 on camshaft 140, the pumping elements may be sequentially urged against the tubing to create a moving zone of occlusion along the length the tubing during revolution of camshaft 140.

According to various implementations of the subject technology, the camshaft 140 is configured (e.g., fitted) with a test cam 142. As will be described further test cam 142 may be configured to freely rotate around the camshaft 140 and, when activated, to lock in place on the camshaft and to move with the camshaft to activate a pumping element 74 associated with the test cam 142, thereby causing the pumping element to compress the intermediate portion of the tubing. In some implementations, the pumping element associated with the test cam 142 may be one of a set of intermediate fingers 74, as described, with regard to FIG. 1. The pumping element associated with the test cam 142 may be configured to operate similar to, or in accordance with the example of FIG. 3.

According to various implementations, the test cam 142 includes a needle roller bearing clutch 144 that is press-fit on the cam 142, which then rides on the camshaft 140, as depicted in the example of FIGS. 4A and 4B. The needles in the bearing clutch may roll freely in one direction, but lock to transmit torque when the rotation of the shaft is reversed. In some implementations, such as the depicted example, the test cam 142 is placed between the (e.g., third) torque finger cam and the (e.g., fourth) lower main pumping finger cam. In some implementations, such as the depicted example, the test cam 140 is configured with a shape that produces a stroke of approximately 0.01".

FIGS. 5A and 5B depict examples of rotation and engagement of the test cam 142, including the one way bearing clutch, for testing a pumping element, according to various aspects of the subject technology. Under normal pumping operation, when the cam is not in use, the test cam 142 may rotate freely and may be biased to park up against the upper frame 138 so that it does not affect the pumping operation. A small torsion spring (not shown) may be incorporated to ensure that the cam remains in this position until it is needed to perform a test, as will be described further.

While in a test mode, the test cam 142 is configured to displace a pumping element between upper and lower occluder elements 72, 76-in the depicted example, the main pumping finger-causing the fluid pressure to rise in the captured volume during the portion of the cam cycle where both upper and lower occluder fingers are closed. Any leakage can then be detected by the pressure sensors.

As described previously, test cam 142 may include a one-way locking bearing that causes it to spin freely when the camshaft 140 is rotating in a first direction 200, and to lock when the camshaft 140 rotates in an opposite direction 202. While in pumping mode the camshaft is rotating in the first direction 200 where the bearing is freewheeling. Test cam 142 includes a flange 204 that projects outward from its center. Test cam 142 may spin freely but may follow the cam motion until flange is moved to park up against the upper frame 138, as shown in FIG. 5A. The flange 204 includes a ridge or stop 206 that parks up against the upper frame 138.

When the infusion device 10 is placed in a test mode, the motor may reverse the camshaft 140 to rotate, locking the bearing thereby causing the camshaft 140 to transmit torque to the cam 142 making it turn in the opposite direction 202. When the cam 142 is further turned by way of being locked to the motion of the camshaft 140, it pushes out on the pumping finger 208, as shown in FIG. 5B.

After a fill portion of the cycle, both occluder elements 72, 76 may be closed and fluid trapped within a portion of intermediate section of the fluid tubing 66 by way of the occluders. At this time the pumping chamber (tubing) may be filled with fluid. According to various implementations, there is a first rotational distance (e.g., approximately 19°) of cam rotation until the lower occluder 76 begins to open to begin the delivery portion of the cycle. During this portion of the cam cycle, the subject technology may perform a check on the occluders 72, 76 to verify they are sealing as expected. This may be done by rotating the cam in the opposite direction for a second rotational distance (e.g., approximately 15°). The test cam 142 will then push on the pumping finger 208 causing the fluid pressure to rise in the closed control volume. If there is any leakage, then a pressure rise may be seen by the upstream pressure sensor 80 when the upper occluder 72 a is leaking, the downstream sensor 82 with the lower occluder 76 leaking, or both sensors if both occluders are leaking. If no leakage is detected, then the pumping may continue as expected.

The test can be performed prior to beginning an infusion when the door 50 is closed and the pump camshaft 140 moves to the home position. It can also be performed intermittently during an infusion to not significantly disrupt the flow continuity.

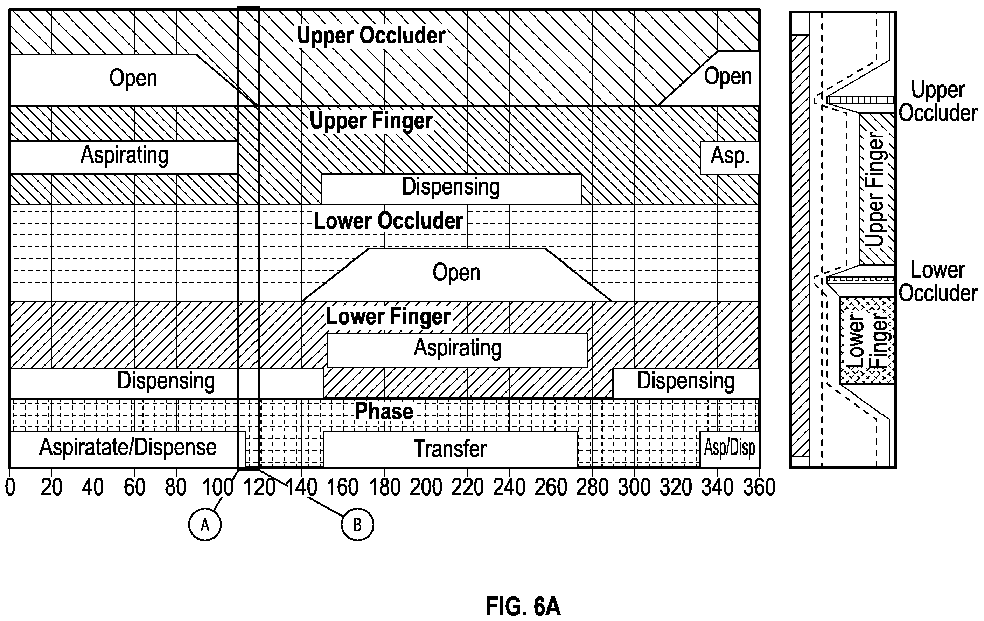

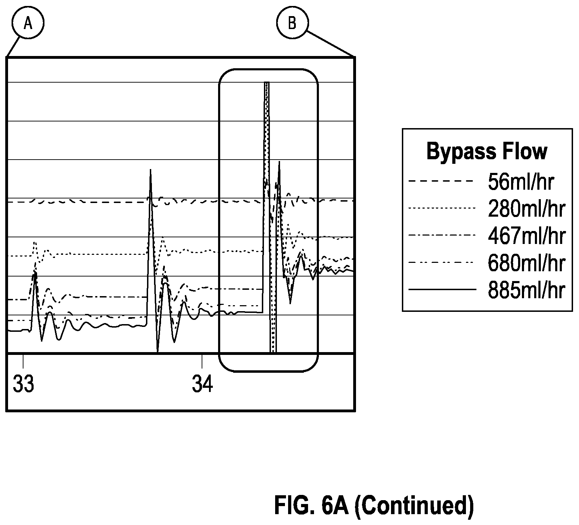

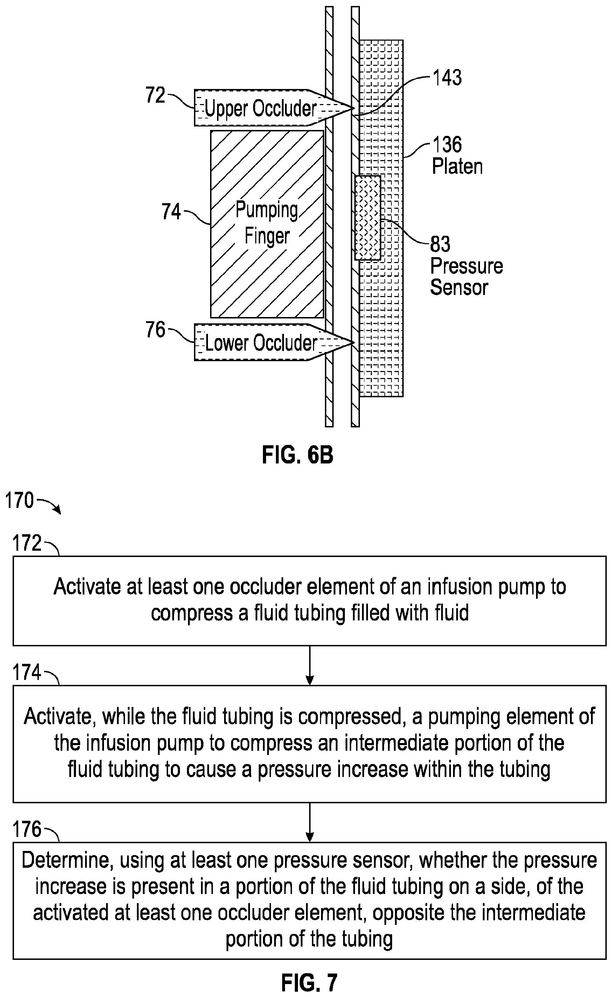

FIG. 6A depicts an example cam phase diagram for testing a pumping element, according to various aspects of the subject technology. Each zone depicted in FIG. 6 corresponds to a pumping element 72, 74, 76, 78, and illustrates an example pumping function of the element according to a complete cam rotation (360°). That is which pumping elements are closing (compressing the tube) and which elements are opening, thereby creating an aspiration phase and a dispensing phase.

In the depicted example, initially, from 0° to 90°, the upper occluder 72 is open, while the lower occluder remains closed. The upper occluder 72 completes closing at about 120°; however, the fluid tubing may be sufficiently compressed to stop aspiration of the fluid at about 110°. The lower occluder remains closed until the cam reaches 140°. While the upper occluder 72 is open-until it begins to close at about 90°-the upper finger 74 is aspirating. The upper occluder closes between 90-120°, and the lower occluder begins to open at 140°. At this point, the lower finger will begin to deliver the fluid. In the depicted example, delivery begins at about 145°, with about 5° rotation accounting for the time to decompress the tubing.

The stepper motor actuates the pumping finger which creates a signal in the line which is picked up by the pressure sensors 80, 82. Upper pressure sensor 80 signals are depicted (below the cam rotation diagram) for the example rotation between 110° and 120°, wherein the upper occluder 72 is not completely closed, yet the tube is sufficiently compressed such that aspiration is ended. Each depicted signal is for a different flow rate as the upper occluder 72 fully closes.

According to various aspects of the subject technology, when both the upstream and downstream occluders 72, 76 are closed, for example between 120° and 140° rotation, the pump 10 may be switched to a test mode in which the cam is rotated in the opposite direction to move the test cam 142 against the pumping finger 74, causing a pressure increase within the tubing between the two occluders. When the occluders 72, 76 are operating normally, and both are closed, there should not be any pressure detection by pressure sensors 80, 82. If there is a gap, even if small, a signal transient will be detected.

According to some implementations, the back rotation of the cam is less than the rotation between the closing and opening of the upper and lower occluders. In other words, the degrees of cam rotation in the opposite direction is less than the degrees of cam rotation in the forward direction while both occluders remain close. In the depicted example, both occluders are closed between 120° and 140° rotation and between 290° and 310° rotation, about a 20° difference in each case. Accordingly, the test cam 142 (including e.g., the size of flange 204) may be configured such that a back rotation of 15° causes the pumping element 208 to move outward against the tubing. Since the other cam lobes 150 continuously move with movement of the camshaft 140, the motion to activate the test cam 142 is narrowed so that an occluder (e.g., the upper occluder 72) is not opened during the test procedure due to the back rotation of the cam. In some implementations, during the test procedure the camshaft 140 may be rotated back and forth within the narrow band of rotation to apply a pumping force against the tubing while the occluders are closed (e.g., the camshaft may be rotated back and forth between 122° and 137°).

The process can be part of a system check when the pump starts up. In some implementations, the process may be initiated (after the line is primed) responsive to detection of an error by the pump.

FIG. 6B depicts an example sensor positioning for testing a pumping element, according to various aspects of the subject technology. In the depicted example, a pressure sensor 83 is positioned in the platen 136 or on the pumping figure 74, between the upper occluder element 72 and the lower occluder element 76. According to various implementations, the pressure sensor 83 may detect a faulty occluder in the same way as described previously, with the exception that the processor of the system may configured to use the pressure sensor 83 to detect pressure loss in the captured volume of fluid (between occluder elements 72, 76) which would then indicate a leaking occluder finger.

Accordingly, the system may activate an upper occluder element 72 and a lower occluder element 76 of the infusion pump 10 to compress a fluid tubing 66 filled with a fluid to isolate the fluid in an intermediate portion of the fluid tubing between the upper occluder element 72 and the lower occluder element 76 from other portions of the fluid tubing. While the fluid tubing is compressed by the occluder elements, a pumping element (e.g., test cam 142) may be activated to compress the intermediate portion of the fluid tubing to cause a pressure increase within the intermediate portion of the fluid tubing. Using the pressure sensor 83, the system then determines whether a pressure decrease is present in the intermediate portion of the tubing. If a pressure decrease is detected then the pump (e.g., one of the occluder elements 72, 76) may be determined to be faulty.

FIG. 7 depicts an example process 170 for testing a pumping element, according to aspects of the subject technology. For explanatory purposes, the various blocks of example process 170 are described herein with reference to FIGS. 1 through 6, and the components and/or processes described herein. The one or more of the blocks of process 170 may be implemented, for example, by one or more computing devices including, for example, within infusion device 10. In some implementations, one or more of the blocks may be implemented based on one or more machine learning algorithms. In some implementations, one or more of the blocks may be implemented apart from other blocks, and by one or more different processors or devices. Further for explanatory purposes, the blocks of example process 170 are described as occurring in serial, or linearly. However, multiple blocks of example process 170 may occur in parallel. In addition, the blocks of example process 70 need not be performed in the order shown and/or one or more of the blocks of example process 70 need not be performed.

In the depicted example, at least one occluder element of an infusion pump is activated to compress a fluid tubing filled with a fluid, to isolate the fluid in an upstream portion of the fluid tubing from a downstream portion of the tubing (172). According to various implementations, the infusion pump 10 includes an upper occluder element 72 configured to compress an upstream portion of the fluid tubing, and a lower occluder element 76 configured to compress a downstream portion of the fluid tubing. In this regard, the upper occluder element 72 may be activated to compress an upstream portion of the fluid tubing together with activation of the lower occluder element to compress a downstream portion of the fluid tubing while the upstream portion is compressed.

While the fluid tubing is compressed by the occluder element(s), a pumping element of the infusion pump is activated to compress an intermediate portion of the fluid tubing, between the downstream portion and the upstream portion, to cause a pressure increase within the intermediate portion of the fluid tubing (174). While both occluder elements 72, 76 are activated, the intermediate portion of the fluid tubing may be compressed between the compressed upstream portion and the compressed downstream portion.

Using at least one pressure sensor, the processor of the infusion pump 10 determines whether the pressure increase is present in a portion of the fluid tubing on a side, of the activated at least one occluder element, opposite the intermediate portion of the tubing (176). According to various implementations, the infusion pump 10 may include an upstream pressure sensor 80 and a downstream pressure sensor 82. In this regard, both may be used to detect whether the pressure increase. The upstream pressure sensor, located upstream of the upper occluder element may be used to detect pressure fluctuations above the upper occluder element, and the downstream pressure sensor, located downstream of the lower occluder element may be used to detect pressure fluctuations below the lower occluder element.

In some implementations, the pumping element 74 is activated to compress the intermediate portion of the tubing by causing a camshaft 140 of the infusion pump 10 to rotate to cause the upper occluder element and the lower occluder element to open and close independently according to a predetermined pattern that corresponds to a degree of rotation of the camshaft. Also, a cam 142, that is coupled to the camshaft and configured to freely rotate around the camshaft, may be caused to lock in place on the camshaft and to move with the camshaft to activate the pumping element, thereby causing the pumping element to compress the intermediate portion of the tubing.

In some implementations, the cam 142 is caused to lock in place comprises reversing a direction that the camshaft is rotating. In this regard, the camshaft 140 may be rotated in a forward direction for a first predetermined degrees of cam rotation, after both upper occluder element and the lower occluder element are activated, to compress the fluid tubing. The pump and the camshaft (and the cam lobes) are configured so that this first predetermined degrees of cam rotation does not cause a decompression of the fluid tubing. Then, while both occluders remain close, the camshaft may be caused to rotate in a reverse direction that is less than the first predetermined degrees of cam rotation in the forward direction. As shown in FIG. 5B, this reverse rotation causes the pumping element 74, 208 to compress the tubing.

In some implementations, the rotating of the camshaft may be alternated in the forward direction and in the reverse direction multiple times, with each rotation not exceeding the first predetermined degrees of cam rotation. The pressure increase may then be detected at any time during each rotation, for example, by pressure sensors 80, 82.

According to some implementations, the infusion pump 10 may include maintenance mode that may be activated, for example, by a control on the pump or on a device connected to the pump. In this regard, the processor of the infusion device may receive an indication to enter a pump maintenance mode (e.g., via selection of the control by a user). Responsive to receiving the indication, the pump processor may automatically activate the upper and lower occlusion elements, and the pumping element of the infusion pump to compress an intermediate portion of the tubing, and automatically activate the upstream and downstream pressure sensors to detect whether the pressure increase is present.

According to various implementations, detection of an increase in pressure at a pressure sensor 80, 82 may indicate fault of the occluder element 72, 76 near the pressure sensor. In some implementations, an indication of the faulty occluder element may be provided for display by a display device, such as a display of the pump 10, or a computing device connected to the pump. For example, based on detecting the pressure increase by the upstream pressure sensor, an indication that the upper occluder element is faulty may be provided and displayed. Similarly, based on detecting the pressure increase by the downstream pressure sensor, an indication that the lower occluder element is faulty may be provided and displayed.

Many of the above-described devices, systems and methods, may also be implemented as software processes that are specified as a set of instructions recorded on a computer readable storage medium (also referred to as computer readable medium), and may be executed automatically (e.g., without user intervention). When these instructions are executed by one or more processing unit(s) (e.g., one or more processors, cores of processors, or other processing units), they cause the processing unit(s) to perform the actions indicated in the instructions. Examples of computer readable media include, but are not limited to, CD-ROMs, flash drives, RAM chips, hard drives, EPROMs, etc. The computer readable media does not include carrier waves and electronic signals passing wirelessly or over wired connections.

The term "software" is meant to include, where appropriate, firmware residing in read-only memory or applications stored in magnetic storage, which can be read into memory for processing by a processor. Also, in some implementations, multiple software aspects of the subject disclosure can be implemented as sub-parts of a larger program while remaining distinct software aspects of the subject disclosure. In some implementations, multiple software aspects can also be implemented as separate programs. Finally, any combination of separate programs that together implement a software aspect described here is within the scope of the subject disclosure. In some implementations, the software programs, when installed to operate on one or more electronic systems, define one or more specific machine implementations that execute and perform the operations of the software programs.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

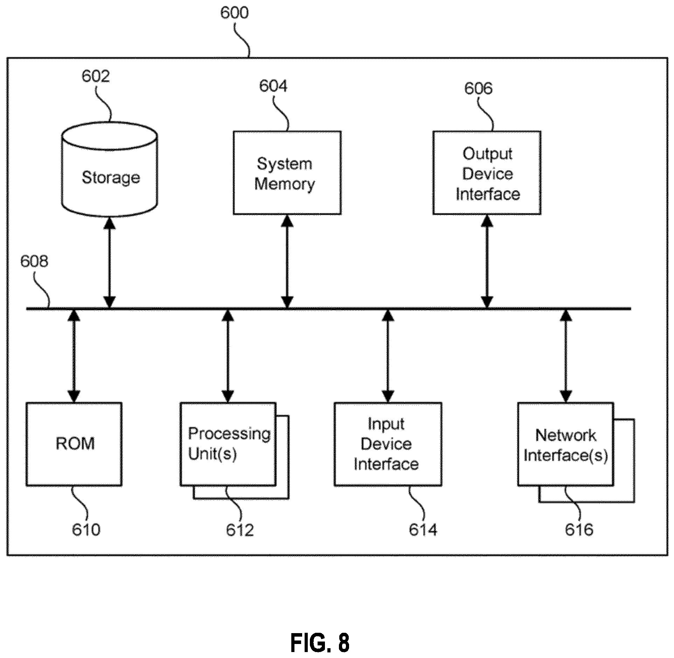

FIG. 8 is a conceptual diagram illustrating an example electronic system 600 for testing a pumping element, according to aspects of the subject technology. Electronic system 600 may be a computing device for execution of software associated with one or more components and processes provided by FIGS. 1 to 7, including but not limited to infusion device 10. Electronic system 600 may be representative of a device used in connection or combination with the disclosure regarding FIGS. 1 to 7. In this regard, electronic system 600 may be a device connected to the infusion device 10, for example, to activate the occluders and/or pumping fingers, the cam 142, or to display results of the tests described herein. For example, system 600 may be representative of a personal computer or a mobile device such as a smartphone, tablet computer, laptop, PDA, an augmented reality device, a wearable such as a watch or band or glasses, or combination thereof, or other touch screen or television with one or more processors embedded therein or coupled thereto, or any other sort of computer-related electronic device having network connectivity.

Electronic system 600 may include various types of computer readable media and interfaces for various other types of computer readable media. In the depicted example, electronic system 600 includes a bus 608, processing unit(s) 612, a system memory 604, a read-only memory (ROM) 610, a permanent storage device 602, an input device interface 614, an output device interface 606, and one or more network interfaces 616. In some implementations, electronic system 600 may include or be integrated with other computing devices or circuitry for operation of the various components and processes previously described.

Bus 608 collectively represents all system, peripheral, and chipset buses that communicatively connect the numerous internal devices of electronic system 600. For instance, bus 608 communicatively connects processing unit(s) 612 with ROM 610, system memory 604, and permanent storage device 602.

From these various memory units, processing unit(s) 612 retrieves instructions to execute and data to process, in order to execute the processes of the subject disclosure. The processing unit(s) can be a single processor or a multi-core processor in different implementations.

ROM 610 stores static data and instructions that are needed by processing unit(s) 612 and other modules of the electronic system. Permanent storage device 602, on the other hand, is a read-and-write memory device. This device is a non-volatile memory unit that stores instructions and data even when electronic system 600 is off. Some implementations of the subject disclosure use a mass-storage device (such as a magnetic or optical disk and its corresponding disk drive) as permanent storage device 602.

Other implementations use a removable storage device (such as a floppy disk, flash drive, and its corresponding disk drive) as permanent storage device 602. Like permanent storage device 602, system memory 604 is a read-and-write memory device. However, unlike storage device 602, system memory 604 is a volatile read-and-write memory, such as random access memory. System memory 604 stores some of the instructions and data that the processor needs at runtime. In some implementations, the processes of the subject disclosure are stored in system memory 604, permanent storage device 602, and/or ROM 610. From these various memory units, processing unit(s) 612 retrieves instructions to execute and data to process, in order to execute the processes of some implementations.

Bus 608 also connects to input and output device interfaces 614 and 606. Input device interface 614 enables the user to communicate information and select commands to the electronic system. Input devices used with input device interface 614 include, e.g., alphanumeric keyboards and pointing devices (also called "cursor control devices"). Output device interfaces 606 enables, e.g., the display of images generated by the electronic system 600. Output devices used with output device interface 606 include, e.g., printers and display devices, such as cathode ray tubes (CRT) or liquid crystal displays (LCD). Some implementations include devices such as a touchscreen that functions as both input and output devices.

Also, as shown in FIG. 8, bus 608 also couples electronic system 600 to a network (not shown) through network interfaces 616. Network interfaces 616 may include, e.g., a wireless access point (e.g., Bluetooth or WiFi) or radio circuitry for connecting to a wireless access point. Network interfaces 616 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 600 can be used in conjunction with the subject disclosure.

These functions described above can be implemented in computer software, firmware, or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs). In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; e.g., by sending web pages to a web browser on a user's client device in response to requests received from the web browser.

Implementations of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some implementations, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The term website, as used herein, may include any aspect of a website, including one or more web pages, one or more servers used to host or store web related content, etc. Accordingly, the term website may be used interchangeably with the terms, web page and server. As used herein a "user interface" (also referred to as an interactive user interface, a graphical user interface or a UI) may refer to a network based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, web services, or rich site summary (RSS). In some implementations, a UI may be included in a stand-alone client (for example, thick client, fat client) configured to communicate (e.g., send or receive data) in accordance with one or more of the aspects described. The communication may be to or from a medical device, diagnostic device, monitoring device, or server in communication therewith.

The predicate words "configured to", "operable to", and "programmed to" do not imply any particular tangible or intangible modification of a subject, but, rather, are intended to be used interchangeably. For example, a processor configured to monitor and control an operation or a component, may also mean the processor being programmed to monitor and control the operation or the processor being operable to monitor and control the operation. Likewise, a processor configured to execute code can be construed as a processor programmed to execute code or operable to execute code.

The term automatic, as used herein, may include performance by a computer or machine without user intervention; for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

As used herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "implementation" does not imply that such implementation is essential to the subject technology or that such implementation applies to all configurations of the subject technology. A disclosure relating to an implementation may apply to all implementations, or one or more implementations. An implementation may provide one or more examples. A phrase such as an "implementation" may refer to one or more implementations and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

## Claims

1. An infusion pump (10), comprising:
a pumping mechanism (70) comprising a lower occluder element (76, 130) configured to compress a downstream portion of a fluid tubing (103) filled with a fluid to isolate the fluid in an upstream portion of the fluid tubing from the downstream portion of the fluid tubing (103) when the fluid tubing (103) is received by the infusion pump (10);
a pumping element (72, 74, 76, 78) configured to compress an intermediate portion of the tubing, between the downstream portion and the upstream portion;
a downstream pressure sensor (82) located downstream of the lower occluder element (76, 130); and
a processor configured to serially:
activate the lower occluder element (76, 130) of the infusion pump (10) to compress the fluid tubing (103) filled with the fluid to isolate the fluid in the upstream portion of the tubing from the downstream portion of the tubing; and
while the fluid tubing (103) is compressed by the lower occluder element (76, 130):
cause the pumping element (72, 74, 76, 78) to compress the intermediate portion of the fluid tubing (103), between the downstream portion and the upstream portion, to cause a pressure increase within the intermediate portion of the fluid tubing(103) above the activated lower occluder element (76, 130); and
detect, using the downstream pressure sensor (82), when the pressure is increased within the intermediate portion of the fluid tubing (103) above the activated lower occluder element (76, 130), whether a pressure increase is present in the downstream portion of the fluid tubing (103) below the activated lower occluder element (76, 130), opposite the intermediate portion of the tubing.

2. The infusion pump (10) of Claim 1, further comprising:
an upper occluder element (72, 130) configured to compress an upstream portion of the fluid tubing (103);
wherein activating the lower occluder element (76, 130) comprises:
activating the upper occluder element (72, 130) to compress an upstream portion of the fluid tubing (103); and
activating the lower occluder element (76, 130) to compress a downstream portion of the fluid tubing (103) while the upstream portion is compressed, wherein the intermediate portion of the fluid tubing (103) is between the compressed upstream portion and the compressed downstream portion.

3. The infusion pump (10) of Claim 2, further comprising:
an upstream pressure sensor (80), located upstream of the upper occluder element (72, 130); and
determining whether the pressure increase is present using the upstream pressure sensor (80) of the infusion pump (10), and using the downstream pressure sensor (82) of the infusion pump (10).

4. The infusion pump of any one of Claim 3, wherein the infusion pump (10) further comprises:
a camshaft (140) configured to cause the upper occluder element (72, 130) and the lower occluder element (76, 130) to open and close independently according to a predetermined pattern that corresponds to a degree of rotation of the camshaft (140); and
a cam (132) coupled to the camshaft (140) and configured to freely rotate around the camshaft (140),
wherein causing the pumping element (72, 74, 76, 78) of the infusion pump (10) to compress the intermediate portion of the tubing comprises:
causing the camshaft (140) of the infusion pump (10) to rotate; and
causing the cam (132) coupled to the camshaft (140) to lock in place on the camshaft (140) and to move with the camshaft (140) to activate the pumping element (72, 74, 76, 78), thereby causing the pumping element (72, 74, 76, 78) to compress the intermediate portion of the tubing.

5. The infusion pump (10) of Claim 4, wherein the processor is further configured to:
reverse a direction that the camshaft (140) is rotating to lock the cam (132) in place.

6. The infusion pump (10) of Claim 5, wherein the processor is further configured to:
cause the camshaft (140) to rotate in a forward direction for a first predetermined degrees of cam rotation after both upper occluder element (72, 130) and the lower occluder element (76, 130) are activated to compress the fluid tubing (103), the first predetermined degrees of cam rotation not causing a decompression of the fluid tubing (103); and
cause, while both occluders remain close, the camshaft (140) to rotate in a reverse direction that is less than the first predetermined degrees of cam rotation in the forward direction.

7. The infusion pump (10) of Claim 6, wherein the processor is further configured to:
alternate the rotating of the camshaft (140) in the forward direction and in the reverse direction multiple times, each rotation not exceeding the first predetermined degrees of cam rotation; and
determine whether the pressure increase is present during each rotation.

8. The infusion pump (10) of any one of Claims 3 through 7, wherein the processor is further configured to:
receive an indication to enter a pump maintenance mode; and
responsive to receiving the indication:
automatically activate by the infusion pump (10) the upper and lower occlusion elements, and the pumping element (72, 74, 76, 78) of the infusion pump (10) to compress an intermediate portion of the tubing; and
automatically activate the upstream and downstream pressure sensors (80, 82) to detect whether the pressure increase is present.

9. The infusion pump (10) of Claim 8, wherein the processor is further configured to:
detect the pressure increase by the upstream pressure sensor (80); and
based on detecting the pressure increase by the upstream pressure sensor (80), provide an indication that the upper occluder element (72, 130) is faulty.

10. The infusion pump (10) of Claim 8, further comprising:
detect the pressure increase by the downstream pressure sensor (82); and
based on detecting the pressure increase by the downstream pressure sensor (82), provide an indication that the lower occluder element (76, 130) is faulty.

11. A method, comprising:
activating a lower occluder element (76, 130) of an infusion pump (10) to compress a downstream portion of a fluid tubing (103) filled with a fluid to isolate the fluid in an upstream portion of the fluid tubing (103) from a downstream portion of the tubing; and
while the fluid tubing (103) is compressed by the lower occluder element (76, 130):
causing a pumping element (72, 74, 76, 78) of the infusion pump (10) to compress an intermediate portion of the fluid tubing (103), between the downstream portion and the upstream portion, to cause a pressure increase within the intermediate portion of the fluid tubing (103) above the activated lower occluder element (76, 130); and
detect, using a downstream pressure sensor (82) located downstream of the lower occluder element (76, 130), when the pressure is increased within the intermediate portion of the fluid tubing (103) above the activated lower occluder element (76, 130), whether a pressure increase is present in the downstream portion of the fluid tubing (103) below the activated lower occluder element (76, 130), opposite the intermediate portion of the tubing.

12. The method of Claim 11, wherein activating the lower occluder element (76, 130) comprises:
activating an upper occluder element (72, 130) to compress an upstream portion of the fluid tubing (103); and
activating the lower occluder element (76, 130) to compress a downstream portion of the fluid tubing (103) while the upstream portion is compressed, wherein the intermediate portion of the fluid tubing (103) is between the compressed upstream portion and the compressed downstream portion.

13. The method of Claim 12, further comprising:
determining whether the pressure increase is present using an upstream pressure sensor (80) of the infusion pump (10), located upstream of the upper occluder element (72, 130), and using the downstream pressure sensor (82) of the infusion pump (10), located downstream of the lower occluder element (76, 130), and wherein causing the pumping element (72, 74, 76, 78) of the infusion pump (10) to compress the intermediate portion of the tubing comprises:
causing a camshaft (140) of the infusion pump (10) to rotate to cause the upper occluder element (72, 130) and the lower occluder element (76, 130) to open and close independently according to a predetermined pattern that corresponds to a degree of rotation of the camshaft (140); and
causing a cam (132), that is coupled to the camshaft (140) and configured to freely rotate around the camshaft (140), to lock in place on the camshaft (140) and to move with the camshaft (140) to activate the pumping element (72, 74, 76, 78), thereby causing the pumping element (72, 74, 76, 78) to compress the intermediate portion of the tubing.

14. The method of Claim 13, wherein causing the cam (132) to lock in place comprises reversing a direction that the camshaft (140) is rotating, and wherein reversing the direction that the camshaft (140) is rotating comprises:
rotating the camshaft (140) in a forward direction for a first predetermined degrees of cam rotation after both upper occluder element (72, 130) and the lower occluder element (76, 130) are activated to compress the fluid tubing (103), the first predetermined degrees of cam rotation not causing a decompression of the fluid tubing (103); and
causing, while both occluders remain close, the camshaft (140) to rotate in a reverse direction that is less than the first predetermined degrees of cam rotation in the forward direction.

15. A non-transitory computer-readable medium comprising instructions that, when executed by a processor, cause an infusion device to perform the method of any one of Claims 11 through 14.

## Patentansprüche

1. Infusionspumpe (10), umfassend:
einen Pumpmechanismus (70), der ein unteres Absperrelement (76, 130) umfasst, das so ausgebildet ist, dass es einen stromabwärts gelegenen Abschnitt eines mit einem Fluid gefüllten Fluidschlauchs (103) zusammendrückt, um das Fluid in einem stromaufwärts gelegenen Abschnitt des Fluidschlauchs vom stromabwärts gelegenen Abschnitt des Fluidschlauchs (103) zu trennen, wenn der Fluidschlauch (103) von der Infusionspumpe (10) aufgenommen wird;
ein Pumpelement (72, 74, 76, 78), das so ausgelegt ist, dass es einen Zwischenabschnitt des Schlauchs zwischen dem stromabwärtigen Abschnitt und dem stromaufwärtigen Abschnitt zusammendrückt;
einen stromabwärtigen Drucksensor (82), der stromabwärts des unteren Absperrelements (76, 130) angeordnet ist; und
einen Prozessor, der so ausgelegt ist, dass er nacheinander ausführt:
Aktivieren des untere Absperrelements (76, 130) der Infusionspumpe (10), um den mit der Flüssigkeit gefüllten Flüssigkeitsschlauch (103) zusammenzudrücken und so die Flüssigkeit im stromaufwärtigen Abschnitt des Schlauchs vom stromabwärtigen Abschnitt des Schlauchs zu trennen; und
während der Flüssigkeitsschlauch (103) durch das untere Absperrelement (76, 130) zusammengedrückt wird:
Veranlassen, dass das Pumpelement (72, 74, 76, 78) den mittleren Abschnitt des Fluidschlauchs (103) zwischen dem stromabwärts gelegenen Abschnitt und dem stromaufwärts gelegenen Abschnitt zusammenzudrückt, um einen Druckanstieg innerhalb des mittleren Abschnitts des Fluidschlauchs (103) oberhalb des aktivierten unteren Absperrelements (76, 130) zu bewirken; und
Erfassen mithilfe des stromabwärts gelegenen Drucksensors (82), wenn der Druck im mittleren Abschnitt des Fluidschlauchs (103) ansteigt oberhalb des aktivierten unteren Verschlusselements (76, 130), ob ein Druckanstieg im stromabwärtigen Abschnitt des Fluidschlauchs (103) unterhalb des aktivierten unteren Verschlusselements (76, 130) vorliegt, gegenüber dem mittleren Abschnitt des Schlauchs.

2. Infusionspumpe (10) nach Anspruch 1, ferner umfassend:
ein oberes Absperrelement (72, 130), das so ausgebildet ist, dass es einen stromaufwärtigen Abschnitt des Fluidschlauchs (103) zusammendrückt;
wobei das Aktivieren des unteren Absperrelements (76, 130) umfasst:
Aktivieren des oberen Absperrelements (72, 130), um einen stromaufwärtigen Abschnitt des Fluidschlauchs (103) zusammenzudrücken; und
Aktivieren des unteren Absperrelements (76, 130), um einen stromabwärts gelegenen Abschnitt des Fluidschlauchs (103) zusammenzudrücken, während der stromaufwärts gelegene Abschnitt zusammengedrückt ist, wobei sich der mittlere Abschnitt des Fluidschlauchs (103) zwischen dem zusammengedrückten stromaufwärts gelegenen Abschnitt und dem zusammengedrückten stromabwärts gelegenen Abschnitt befindet.

3. Infusionspumpe (10) nach Anspruch 2, ferner umfassend:
einen stromaufwärts gelegenen Drucksensor (80), der stromaufwärts des oberen Absperrelements (72, 130) angeordnet ist; und
Feststellen, ob der Druckanstieg vorliegt, unter Verwendung des stromaufwärts gelegenen Drucksensors (80) der Infusionspumpe (10) und unter Verwendung des stromabwärts gelegenen Drucksensors (82) der Infusionspumpe (10).

4. Infusionspumpe nach einem der Ansprüche 3, wobei die Infusionspumpe (10) ferner umfasst:
eine Nockenwelle (140), die so ausgelegt ist, dass sie bewirkt, dass sich das obere Verschlusselement (72, 130) und das untere Verschlusselement (76, 130) unabhängig voneinander gemäß einem vorgegebenen Muster öffnen und schließen, das einem Drehwinkel der Nockenwelle (140) entspricht; und
einen Nocken (132), die mit der Nockenwelle (140) gekoppelt und so ausgebildet ist, dass sie sich frei um die Nockenwelle (140) dreht,
wobei das Veranlassen des Pumpelements (72, 74, 76, 78) der Infusionspumpe (10) zum Zusammendrücken des Zwischenabschnitts des Schlauchs Folgendes umfasst:
Drehen der Nockenwelle (140) der Infusionspumpe (10) bewirken; und
Verriegeln des mit der Nockenwelle (140) gekoppelten Nockens (132) an der Nockenwelle (140) sowie dessen Mitbewegung mit der Nockenwelle (140) bewirken, um das Pumpelement (72, 74, 76, 78) zu aktivieren, wodurch das Pumpelement (72, 74, 76, 78) den Zwischenabschnitt des Schlauchs komprimiert.

5. Infusionspumpe (10) nach Anspruch 4, wobei der Prozessor ferner so konfiguriert ist, dass er ausführt:
Umkehren Drehrichtung der Nockenwelle (140), um den Nocken (132) in seiner Position zu arretieren.

6. Infusionspumpe (10) nach Anspruch 5, wobei der Prozessor ferner so konfiguriert ist, dass er ausführt:
Veranlassen der Nockenwelle (140), sich um einen ersten vorbestimmten Winkel der Nockenrotation in Vorwärtsrichtung zu drehen, nachdem sowohl das obere Absperrelement (72, 130) als auch das untere Absperrelement (76, 130) aktiviert wurden, um den Fluidschlauch (103) zusammenzudrücken, wobei die ersten vorbestimmten Nockenrotationsgrade keine Dekompression des Fluidschlauchs (103) bewirken; und
Veranlassen, während beide Verschlusselemente geschlossen bleiben, die Nockenwelle (140) in einer Rückwärtsrichtung um einen Winkel zu drehen, der kleiner ist als die ersten vorbestimmten Nockenrotationsgrade in Vorwärtsrichtung.

7. Infusionspumpe (10) nach Anspruch 6, wobei der Prozessor ferner so konfiguriert ist, dass er ausführt:
Abwechseln der Drehung der Nockenwelle (140) in Vorwärtsrichtung und in Rückwärtsrichtung mehrfach abwechselt, wobei jede Drehung die ersten vorbestimmten Nockendrehwinkel nicht überschreitet; und
Feststellen, ob während jeder Drehung ein Druckanstieg vorliegt.

8. Infusionspumpe (10) nach einem der Ansprüche 3 bis 7, wobei der Prozessor ferner so konfiguriert ist, dass er ausführt:
Empfangen einer Anweisung zum Aufrufen eines Pumpenwartungsmodus; und
als Reaktion auf den Empfang der Anweisung:
automatisches Aktivieren durch die Infusionspumpe (10) des oberen und des unteren Absperrelements sowie des Pumpelements (72, 74, 76, 78) der Infusionspumpe (10), um einen Zwischenabschnitt des Schlauchs zusammenzudrücken; und
automatisches Aktivieren der stromaufwärts und stromabwärts gelegenen Drucksensoren (80, 82), um zu erfassen, ob der Druckanstieg vorliegt.

9. Infusionspumpe (10) nach Anspruch 8, wobei der Prozessor ferner so konfiguriert ist, dass er ausführt:
Erfassen des Druckanstiegs mittels des stromaufwärts gelegenen Drucksensors (80); und
Bereitstellen auf der Grundlage der Erfassung des Druckanstiegs durch den stromaufwärts gelegenen Drucksensor (80) einer Meldung, dass das obere Absperrelement (72, 130) fehlerhaft ist.

10. Infusionspumpe (10) nach Anspruch 8, ferner umfassend:
Erfassen des Druckanstiegs durch den stromabwärts gelegenen Drucksensor (82); und
Bereitstellen auf der Grundlage der Erfassung des Druckanstiegs durch den stromabwärts gelegenen Drucksensor (82) einer Meldung, dass das untere Absperrelement (76, 130) fehlerhaft ist.

11. Verfahren, umfassend:
Aktivieren eines unteren Absperrelements (76, 130) einer Infusionspumpe (10), um einen stromabwärts gelegenen Abschnitt eines mit einem Fluid gefüllten Fluidschlauchs (103) zusammenzudrücken, um das Fluid in einem stromaufwärts gelegenen Abschnitt des Fluidschlauchs (103) von einem stromabwärts gelegenen Abschnitt des Schlauchs zu isolieren; und
während der Fluidschlauch (103) durch das untere Absperrelement (76, 130) zusammengedrückt wird:
Veranlassen eines Pumpelements (72, 74, 76, 78) der Infusionspumpe (10) dazu, einen Zwischenabschnitt des Fluidschlauchs (103) zwischen dem stromabwärtigen Abschnitt und dem stromaufwärtigen Abschnitt zusammenzudrücken, um einen Druckanstieg innerhalb des Zwischenabschnitts des Fluidschlauchs (103) oberhalb des aktivierten unteren Verschlusselements (76, 130) zu bewirken; und
Erfassen, unter Verwendung eines stromabwärts des unteren Absperrelements (76, 130) angeordneten stromabwärts gelegenen Drucksensors (82), wenn der Druck im mittleren Abschnitt des Fluidschlauchs (103) oberhalb des aktivierten unteren Absperrelements (76, 130) erhöht ist, ob im stromabwärts gelegenen Abschnitt der Fluidleitung (103) unterhalb des aktivierten unteren Verschlusselements (76, 130), gegenüber dem Zwischenabschnitt der Leitung, ein Druckanstieg vorliegt.

12. Verfahren nach Anspruch 11, wobei das Aktivieren des unteren Verschlusselements (76, 130) umfasst:
Aktivieren eines oberen Verschlusselements (72, 130), um einen stromaufwärtigen Abschnitt der Fluidleitung (103) zusammenzudrücken; und
Aktivieren des unteren Absperrelements (76, 130), um einen stromabwärts gelegenen Abschnitt des Fluidschlauchs (103) zusammenzudrücken, während der stromaufwärts gelegene Abschnitt zusammengedrückt ist, wobei sich der mittlere Abschnitt des Fluidschlauchs (103) zwischen dem zusammengedrückten stromaufwärts gelegenen Abschnitt und dem zusammengedrückten stromabwärts gelegenen Abschnitt befindet.

13. Verfahren nach Anspruch 12, das ferner umfasst:
Feststellen, ob der Druckanstieg vorliegt, unter Verwendung eines stromaufwärts gelegenen Drucksensors (80) der Infusionspumpe (10), der stromaufwärts des oberen Absperrelements (72, 130) angeordnet ist, und unter Verwendung des stromabwärts gelegenen Drucksensors (82) der Infusionspumpe (10), der stromabwärts des unteren Absperrelements (76, 130) angeordnet ist, und wobei das Veranlassen des Pumpelements (72, 74, 76, 78) der Infusionspumpe (10) dazu zu bringen, den Zwischenabschnitt des Schlauchs zusammenzudrücken, umfasst:
Veranlassen, dass sich eine Nockenwelle (140) der Infusionspumpe (10) dreht, um zu bewirken, dass sich das obere Verschlusselement (72, 130) und das untere Verschlusselement (76, 130) unabhängig voneinander gemäß einem vorgegebenen Muster öffnen und schließen, das einem Drehwinkel der Nockenwelle (140) entspricht; und
Veranlassen, dass ein Nocken (132), das mit der Nockenwelle (140) gekoppelt und so ausgebildet ist, dass es sich frei um die Nockenwelle (140) drehen kann, an der Nockenwelle (140) arretiert wird und sich mit der Nockenwelle (140) bewegt, um das Pumpelement (72, 74, 76, 78) zu bewegen, wodurch das Pumpelement (72, 74, 76, 78) den Zwischenabschnitt des Schlauchs komprimiert.

14. Verfahren nach Anspruch 13, wobei das Verriegeln des Nockens (132) das Umkehren der Drehrichtung der Nockenwelle (140) umfasst, und wobei das Umkehren der Drehrichtung der Nockenwelle (140) umfasst:
Drehen der Nockenwelle (140) in Vorwärtsrichtung um einen ersten vorbestimmten Winkel der Nockenrotation, nachdem sowohl das obere Verschlusselement (72, 130) als auch das untere Verschlusselement (76, 130) aktiviert wurden, um den Fluidschlauch (103) zusammenzudrücken, wobei der erste vorbestimmte Winkel der Nockenrotation keine Dekompression des Fluidschlauchs (103) bewirkt; und
Veranlassen, dass sich die Nockenwelle (140), während beide Verschlusselemente geschlossen bleiben, in einer Rückwärtsrichtung dreht, die um weniger als die ersten vorbestimmten Nocken-Drehwinkel in Vorwärtsrichtung beträgt.

15. Nichtflüchtiges, computerlesbares Medium, das Anweisungen enthält, die, wenn sie von einem Prozessor ausgeführt werden, eine Infusionsvorrichtung veranlassen, das Verfahren gemäß einem der Ansprüche 11 bis 14 durchzuführen.

## Revendications

1. Pompe à perfusion (10), comprenant:
un mécanisme de pompage (70) comprenant un élément d'occlusion inférieur (76, 130) configuré pour comprimer une partie aval d'un tube de fluide (103) rempli d'un fluide afin d'isoler le fluide présent dans une partie amont du tube de fluide de la partie aval du tube de fluide (103) lorsque le tube de fluide (103) est reçu par la pompe à perfusion (10);
un élément de pompage (72, 74, 76, 78) configuré pour comprimer une partie intermédiaire du tube, située entre la partie aval et la partie amont ;
un capteur de pression aval (82) situé en aval de l'élément d'occlusion inférieur (76, 130); et
un processeur configuré pour, successivement :
activer l'élément d'occlusion inférieur (76, 130) de la pompe à perfusion (10) afin de comprimer le tube de fluide (103) rempli de fluide pour isoler le fluide présent dans la partie amont du tube de la partie aval du tube ; et
tandis que le tube de fluide (103) est comprimé par l'élément d'occlusion inférieur (76, 130) :
amener l'élément de pompage (72, 74, 76, 78) à comprimer la partie intermédiaire du tube de fluide (103), située entre la partie aval et la partie amont, afin de provoquer une augmentation de pression au sein de la partie intermédiaire du tube de fluide (103) au-dessus de l'élément d'occlusion inférieur activé (76, 130) ; et
détecter, à l'aide du capteur de pression aval (82), lorsque la pression augmente dans la partie intermédiaire du conduit de fluide (103) au-dessus de l'élément d'occlusion inférieur activé (76, 130), s'il existe une augmentation de pression dans la partie aval du conduit de fluide (103) en dessous de l'élément d'occlusion inférieur activé (76, 130), à l'opposé de la partie intermédiaire du conduit.

2. La pompe à perfusion (10) selon la revendication 1, comprenant en outre :
un élément d'occlusion supérieur (72, 130) configuré pour comprimer une partie amont du tube de fluide (103) ;
dans laquelle l'activation de l'élément d'occlusion inférieur (76, 130) comprend :
l'activation de l'élément d'occlusion supérieur (72, 130) afin de comprimer une partie amont du tuyau de fluide (103) ; et
l'activation de l'élément d'occlusion inférieur (76, 130) pour comprimer une partie aval du tube de fluide (103) tandis que la partie amont est comprimée, la partie intermédiaire du tube de fluide (103) se trouvant entre la partie amont comprimée et la partie aval comprimée.

3. La pompe à perfusion (10) selon la revendication 2, comprenant en outre :
un capteur de pression amont (80), situé en amont de l'élément d'occlusion supérieur (72, 130) ; et
déterminer si l'augmentation de pression est présente à l'aide du capteur de pression amont (80) de la pompe à perfusion (10) et à l'aide du capteur de pression aval (82) de la pompe à perfusion (10).

4. Pompe à perfusion selon l'une quelconque des revendications 3, dans laquelle la pompe à perfusion (10) comprend en outre :
un arbre à cames (140) configuré pour amener l'élément d'occlusion supérieur (72, 130) et l'élément d'occlusion inférieur (76, 130) à s'ouvrir et à se fermer indépendamment selon un schéma prédéterminé qui correspond à un angle de rotation de l'arbre à cames (140) ; et
une came (132) couplée à l'arbre à cames (140) et configurée pour tourner librement autour de l'arbre à cames (140),
dans lequel le fait d'amener l'élément de pompage (72, 74, 76, 78) de la pompe à perfusion (10) à comprimer la partie intermédiaire du tube comprend :
faire tourner l'arbre à cames (140) de la pompe à perfusion (10) ; et
amener la came (132) couplée à l'arbre à cames (140) à se verrouiller en place sur l'arbre à cames (140) et à se déplacer avec l'arbre à cames (140) pour actionner l'élément de pompage (72, 74, 76, 78), amenant ainsi l'élément de pompage (72, 74, 76, 78) à comprimer la partie intermédiaire du tube.

5. La pompe à perfusion (10) selon la revendication 4, dans laquelle le processeur est en outre configuré pour :
inverser le sens de rotation de l'arbre à cames (140) afin de verrouiller la came (132) en place.

6. Pompe à perfusion (10) selon la revendication 5, dans laquelle le processeur est en outre configuré pour :
provoquer la rotation de l'arbre à cames (140) dans le sens avant sur un premier angle prédéterminé de rotation de la came après que l'élément d'occlusion supérieur (72, 130) et l'élément d'occlusion inférieur (76, 130) ont été activés pour comprimer le tube de fluide (103), le premier angle prédéterminé de rotation de la came n'entraînant pas de décompression du tube de fluide (103) ; et
provoquer, tandis que les deux occludeurs restent fermés, la rotation de l'arbre à cames (140) dans le sens inverse, sur un angle inférieur au premier angle prédéterminé de rotation de la came dans le sens avant.

7. La pompe à perfusion (10) selon la revendication 6, dans laquelle le processeur est en outre configuré pour :
alterner plusieurs fois la rotation de l'arbre à cames (140) dans le sens avant et dans le sens arrière, chaque rotation ne dépassant pas les premiers degrés prédéterminés de rotation de la came ; et
déterminer si l'augmentation de pression est présente au cours de chaque rotation.

8. La pompe à perfusion (10) selon l'une quelconque des revendications 3 à 7, dans laquelle le processeur est en outre configuré pour :
recevoir une indication pour passer en mode de maintenance de la pompe ; et
en réponse à la réception de cette indication :
activer automatiquement, par la pompe à perfusion (10), les éléments d'occlusion supérieur et inférieur, ainsi que l'élément de pompage (72, 74, 76, 78) de la pompe à perfusion (10) afin de comprimer une partie intermédiaire du tube ; et
activer automatiquement les capteurs de pression amont et aval (80, 82) afin de détecter si une augmentation de pression est présente.

9. Pompe à perfusion (10) selon la revendication 8, dans laquelle le processeur est en outre configuré pour :
détecter l'augmentation de pression à l'aide du capteur de pression amont (80) ; et
sur la base de la détection de l'augmentation de pression par le capteur de pression amont (80), fournir une indication selon laquelle l'élément d'occlusion supérieur (72, 130) est défectueux.

10. Pompe à perfusion (10) selon la revendication 8, comprenant en outre :
détecter l'augmentation de pression par le capteur de pression en aval (82) ; et
sur la base de la détection de l'augmentation de pression par le capteur de pression aval (82), fournir une indication selon laquelle l'élément d'occlusion inférieur (76, 130) est défectueux.

11. Procédé, comprenant :
l'activation d'un élément d'occlusion inférieur (76, 130) d'une pompe à perfusion (10) afin de comprimer une partie en aval d'un tube de fluide (103) rempli d'un fluide pour isoler le fluide présent dans une partie en amont du tube de fluide (103) de la partie en aval du tube ; et
tandis que le tube de fluide (103) est comprimé par l'élément d'occlusion inférieur (76, 130) :
amener un élément de pompage (72, 74, 76, 78) de la pompe à perfusion (10) à comprimer une partie intermédiaire du tube de fluide (103), située entre la partie aval et la partie amont, afin de provoquer une augmentation de pression à l'intérieur de la partie intermédiaire du tube de fluide (103) au-dessus de l'élément d'occlusion inférieur activé (76, 130) ; et
détecter, à l'aide d'un capteur de pression aval (82) situé en aval de l'élément d'occlusion inférieur (76, 130), si la pression augmente dans la partie intermédiaire du tube de fluide (103) au-dessus de l'élément d'occlusion inférieur activé (76, 130), s'il existe une augmentation de pression dans la partie aval du tube de fluide (103) située en dessous de l'élément d'occlusion inférieur activé (76, 130), à l'opposé de la partie intermédiaire du tube.

12. Procédé selon la revendication 11, dans lequel l'activation de l'élément d'occlusion inférieur (76, 130) comprend :
l'activation d'un élément d'occlusion supérieur (72, 130) afin de comprimer une partie amont du tuyau de fluide (103) ; et
l'activation de l'élément d'occlusion inférieur (76, 130) pour comprimer une partie aval du tube de fluide (103) tandis que la partie amont est comprimée, la partie intermédiaire du tube de fluide (103) se trouvant entre la partie amont comprimée et la partie aval comprimée.

13. Procédé selon la revendication 12, comprenant en outre :
déterminer si l'augmentation de pression est présente à l'aide d'un capteur de pression amont (80) de la pompe à perfusion (10), situé en amont de l'élément d'occlusion supérieur (72, 130), et à l'aide du capteur de pression aval (82) de la pompe à perfusion (10), situé en aval de l'élément d'occlusion inférieur (76, 130), et dans lequel le fait de faire en sorte que les éléments de pompage (72, 74, 76, 78) de la pompe à perfusion (10) à comprimer la partie intermédiaire du tube comprend :
faire tourner un arbre à cames (140) de la pompe à perfusion (10) afin de provoquer l'ouverture et la fermeture indépendantes de l'élément d'occlusion supérieur (72, 130) et de l'élément d'occlusion inférieur (76, 130) selon un schéma prédéterminé qui correspond à un angle de rotation de l'arbre à cames (140) ; et
amener une came (132), qui est couplée à l'arbre à cames (140) et configurée pour tourner librement autour de celui-ci, à se verrouiller en place sur l'arbre à cames (140) et à se déplacer avec celui-ci afin d'activer l'élément de pompage (72, 74, 76, 78), amenant ainsi l'élément de pompage (72, 74, 76, 78) à comprimer la partie intermédiaire du tube.

14. Procédé selon la revendication 13, dans lequel le fait d'amener la came (132) à se verrouiller en place comprend l'inversion du sens de rotation de l'arbre à cames (140), et dans lequel l'inversion du sens de rotation de l'arbre à cames (140) comprend :
faire tourner l'arbre à cames (140) dans le sens avant sur un premier angle prédéterminé de rotation de came après que l'élément d'occlusion supérieur (72, 130) et l'élément d'occlusion inférieur (76, 130) ont tous deux été activés pour comprimer le tube de fluide (103), le premier angle prédéterminé de rotation de came n'entraînant pas de décompression du tube de fluide (103) ; et
provoquer, tandis que les deux éléments d'occlusion restent fermés, la rotation de l'arbre à cames (140) dans un sens inverse sur une amplitude inférieure au premier nombre prédéterminé de degrés de rotation de la came dans le sens avant.

15. Support non éphémère lisible par ordinateur comprenant des instructions qui, lorsqu'elles sont exécutées par un processeur, amènent un dispositif de perfusion à mettre en œuvre le procédé selon l'une quelconque des revendications 11 à 14.
